# EUROPEAN PATENT APPLICATION

(11) **EP 1 149 898 A2**
(43) Date of publication of application: **31.10.2001**
(21) Application number: 01115354.1
(22) Date of filing: 23.12.1994
(51) Int. Cl.: C12N 5/00, C12N 5/06, A01K 67/027

(54) **Embryonic stem cells as nuclear donors and nuclear transfer techniques to produce chimeric and transgenic animals**

(30) Priority: 23.12.1993 US 172385
(62) Divisional of application: 95907932.8
(71) Applicant: Infigen, Inc., Deforest, Wisconsin 53532 (US)
(72) Inventor: Stice, Steven, L., Deforest, WI 53532 (US); Strelchenko, Nick, Deforest, WI 53532 (US); Betthauser, Jeff, Morrisonville, WI 53571 (US); Scott, Brett, Deforest, WI 53571 (US); Jurgella, Gail, Madison, WI 53705 (US)
(74) Representative: Vaillant, Jeanne

(57) **Abstract**

The use of embryonic stem (ES) cells as donor nuclei for nuclear transfer (NT) procedures is provided. The method provides for the production of ES cell derived NT embryos. The use of ES cell derived NT embryos for producing chimeric non-human animals, preferably ungulates is also provided. Additionally, the use of transgenic ES cells as nuclear donors to provide transgenic embryos and transgenic offspring is taught.

## Description

### Field of the Invention

In its broadest embodiment the present invention is directed to the use of embryonic stem (ES) cells as donor nuclei for nuclear transplantation of recipient oocytes.

The present invention is more particularly directed to a method of using ungulate embryonic stem (ES) cells as donor nuclei for nuclear transplantation (NT) of recipient ungulate oocytes.

The present invention is further directed to the use of the resultant ungulate ES cell derived NT embryos for the production of ungulate offspring.

The present invention is also directed to a novel method for producing chimeric ungulate embryos comprising introducing blastomeres obtained from a fertilized embryo into a NT embryo produced using an ES cell as the nuclear donor.

The present invention is further directed to the use of transgenic ES cells as donor nuclei for nuclear transplantation of recipient ungulate recipient oocytes.

The invention is also directed to the use of transgenic ES cell derived NT embryos for the production of transgenic ungulate offspring.

The invention is still further directed to a novel method for the production of transgenic chimeric embryos by combining blastomeres from a fertilized embryo with a transgenic ES cell derived NT embryo.

### Background of the Invention

Embryo multiplication by nuclear transfer, also known as nuclear transplantation (NT), involves the transplantation of living nuclei from embryonic cells, or the whole embryonic cells themselves, into recipient cells, typically unfertilized eggs, followed by the fusion of the donor and recipient. Such transfers are typically made in order to increase the number of genetically identical embryos which can be obtained from elite genetic stock.

The earliest research relating to nuclear transfer of nuclei of embryonic cells into unfertilized eggs was performed in amphibians. Specifically, embryonic frog blastomere cells were separated and the nuclear material was reintroduced into frogs' eggs which had been enucleated. The results showed that adult offspring could be produced by nuclear transfer using blastula cells. See "Transplantation of Living Nuclei from Blastula Cells into Enucleated Frog Egg", Briggs, R., et al., Proc. Natl. Acad. Sci., 38, 455-463, 1972. Subsequent to this research, other experiments were performed in amphibians and amphibian eggs to determine if nuclear material from adult frog somatic or germinal cells could be transplanted into eggs and to ascertain whether the egg would develop into a normal larva. The results showed that adult offspring could not be produced by adult cells. See "Development and Chromosomal Constitution of Nuclear-Transplants Derived from Male Germ Cells," Berradino, MA, et al., J. Exp. Zool., 176, 61-72, 1981.

Transplantation of mammalian living nuclear material into recipient oocytes has also been reported in the literature. The earliest successful reports of nuclear transplantation in higher mammals were achieved using sheep embryos wherein individual blastomeres from 8 and 16 cell embryos were used as the nuclear donors in enucleated or nucleated halves of unfertilized eggs. See "Nuclear Transplantation in Sheep Embryos," Willadsen, S.M. et al., Nature, 320, 64-65, 1986. About the same time, nuclear transplantation of bovine nuclear material was also reported in the literature in "Nuclear Transplantation in Bovine," Robl, J. et al., Theriogenology, 25, 1, 1986. However, the resultant embryos only developed for 43 days out of a normal nine month gestation period.

Fairly recently, substantial improvements in nuclear transplantation methods have been reported in the literature. Many of these improvements have been in the area of enhancing the number of viable embryos which may be obtained from a single fertilized embryo.

Essentially, once a fertilized embryo has reached a cleavage stage, which simply means that the embryo comprises at least two cells, it becomes feasible to transfer the nuclei from the cells, or the entire cells themselves, into recipient oocytes which have been enucleated, to thereby create multiple genetically identical embryos from such fusions. By allowing each of the fused nuclear transfer embryos to develop to a multi-cell stage, and then repeating the nuclear transplantation procedure, it is possible to obtain a number of genetically identical nuclear transfer embryos from a single original donor embryo. Typically blastomeres isolated from pregastrulation embryos comprise the source of such donor nuclei.

An inherent limitation in the commercial use of nuclear transfer processes, as practiced to date, arises from the fact that there exist various inefficiencies in the nuclear transfer process. Most especially, not all of the nuclear fusions result in viable embryos. Also, not all of the embryos produced by nuclear fusion are capable of creating a viable (full term) pregnancy in a surrogate animal. This is evidenced, e.g., by the Robl et al. (Id.) reference discussed supra. Accordingly, substantial effort is currently being directed towards optimizing nuclear transfer techniques at each step of the process, so as to make the overall procedure more economically practical and reproducible.

Two patents which are illustrative of recent improvement in nuclear transplantation methods are U.S. Patent No. 4,994,384 to Prather et al. and U.S. Patent No. 5,057,042 to Massey.

Both of these patents relate to nuclear transplantation methods which enable the serial production of many genetically identical bovine animals. In these nuclear transplantation methods, oocytes are recovered from the ovaries or reproductive tracts of cows. These oocytes are then selected for a particular stage of development, and are enucleated by physical aspiration through a transfer pipette, resulting in an enucleated oocyte which still retains its external membranes and which functions as a recipient donor nucleus or as a donor embryonic cell. Synchronously, a donor embryo at the proper stage of development, typically at the cleavage or morula stage, is manipulated so that one or more cells or blastomeres are removed from the embryo. The donor cell, which, of course, contains its nucleus, is then inserted into the perivitelline space of a recipient oocyte. An electronic pulse is then applied in order to fuse the membranes of the donor cell and the recipient oocyte, thus creating an activated, fused single cell embryo.

The resultant single cell nuclear transfer embryo is then cultured in vitro or in vivo in the oviduct of a mammal, until it reaches a stage of development wherein it may successfully be implanted into a recipient cow. This methodology results in a significant number of fused embryos which are viable, and which when transplanted surgically or non-surgically into the uteri of cows result in viable pregnancies and the production of genetically identical calves.

Since the basic nuclear transfer techniques were set forth in Prather et al. (Id.) and Massey (Id.), various aspects of nuclear transplantation methods have been modified or enhanced. For instance, much is now known about synchronizing and optimizing the cell cycle stage of the donor and recipient cells prior to fusion.

For example, the timing of fusion has recently gained much attention by researchers as being an important variable in the rate of development of the resultant NT embryo to the blastocyst stage. This is significant since until the embryo attains the late morula or blastocyst stage it generally is not feasible to implant the NT embryo into a maternal recipient.

Most of the available evidence suggests that for optimal embryo cloning the oocyte should be activated prior to fusion. Similarly, studies conducted with frog NT embryos have shown that non-activated oocytes provide for the donor cell nuclei (erythrocytes) to be conditioned by the non-activated cytoplasm of the oocyte. However, the resultant NT embryos had to be processed through several serial transfers to maximize the rate of embryonic development. (Orr et al., Proc. Natl. Acad. Sci., USA, 83, 1369-1373, 1986). Moreover, the embryos never were able to attain the adult stage of development, but rather only grew to the tadpole stage. (Id.)

Another aspect of NT methodology which has been substantially improved comprises NT embryo culturing techniques. Culturing methods for fused nuclear transplant embryos have recently been substantially improved enabling the embryo to be cultured in vitro for longer periods of time prior to implantation into maternal recipients. See, e.g., Bondioli et al., "Bovine Nuclear Transplantation," International Application No. PCT/US 88/01906, International Publication No. WO 88/09816, December 15, 1988; and Bondioli et al., Theriogenology, 33, 165-174, 1990 which teach development of bovine embryos to the morula or blastocyst stage in ligated sheet oviduct prior to transfer to maternal recipients, and Bondioli et al., 1990, (Id.) and Bondioli et al., "Bovine Embryo In Vitro Culture," International Publication No. WO 89/07135, (August 10, 1989); which relate to in vitro culture systems for nuclear transfer embryos. Also, cell-free embryo culture, which overcomes the 8-16 cell developmental block, has been reported in U.S. Patent No. 5,096,822 to Rosenkratz, Jr. et al.

Along similar lines, much effort has recently been directed toward maximizing the available supply of genetically identical donor nuclei (or nucleated donor cells) for use in nuclear transfer and for other genetic manipulations. Originally, donor nuclei were obtained directly from embryo cells. However, an inherent limitation to this strategy is that the maximum number of cells available from each embryo is small, on the order of 32-64 cells. It therefore has been the aim of researchers in this field to maximize the number of donor cells from each embryo. This would of course increase the likelihood of obtaining more genetically identical animals from a particular embryo.

Beyond a certain developmental state it is no longer possible to use every embryo cell for nuclear transplantation because some of the cells are no longer totipotent. This occurs because some of the embryonic cells in the embryo have undergone significant cellular differentiation. By contrast, totipotent cells are cells which have not as yet significantly differentiated and thus may be used as donor nuclei or nucleated donor cells in nuclear transplantation. For example, it has been shown that cells of the inner cell mass (ICM), but not trophoblasts, of blastocyst stage bovine embryos (embryo of about 64 cells) may be used as donors for nuclear transfer.

Given the loss of totipotency which starts at about 64 cells into embryonic development, much effort has been directed toward producing continuous cultures of totipotent embryonic cell lines which, under proper growth conditions, remain both totipotent and undifferentiated until these cell lines are induced to differentiate. For example, such stem cell lines have been isolated from mice, and have putatively been obtained from bovine, ovine, hamster, mink and porcine blastocyst ICM cells. (See, e.g., Doetschman et al., "Establishment of hamster blastocyst-derived embryonic stem cells," Devel. Biol., 127, 224-227, 1988; Eistetter, "Pluripotent embryonal stem cells can be established from disaggregated mouse morulae", Devel. Growth and Diff. 31, 275-282, 1989; Flechon et al., "Characterization of ovine and porcine embryonic stem cells", J. Reprod. Ferti., 40, 25, 1990; Matsui et al., "Derivation of pluripotential embryonic stem cells from murine primordial germ cells in culture," Cell, 70, 69, 891-897, 1992; Notarianni et al., "Maintenance and differentiation in culture of pluripotent embryonic cell lines from pig blastocysts," J. Reprod. Fert. Suppl., 43, 255-260, 1990; and Sukoyan et al., "Isolation and culturization of blastocyst-derived stem cell lines from American mink," Mol. Reprod. Devel., 33, 418-437, 1992.

See also, Weima et al., Proceedings 12th Int. Cong. in Animal Reprod., 2, 766-768, 1992, who disclose the isolation of purported permanent embryonic cell lines derived from bovine blastocysts. However, these cell lines were not tested for their ability to differentiate either in vitro or in vivo. Further, Saito et al., Roux's Arch. Dev. Biol., 201, 134-141, 1992, report the isolation of bovine "ES cell-like" cell lines obtained from bovine blastocyst stage embryos which remain viable after several passages.

However, despite the apparent availability of ES cell lines, to the best knowledge of the present inventors, there has not been reported in the literature the successful use of ES cells as nuclear donors for NT. Recently, Tsunoda et al., J. Reproduct. Fertil., 98, 537-540, 1993, reported attempts at utilizing mice ES cells as nuclear donors during NT. The authors disclosed that some of the resultant fusions developed to two-cell, four-cell, morulae and the blastocyst stage. However, upon implantation into maternal recipients, no live fetuses were obtained. Moreover, the authors concluded based on these results that "[t]echniques for the development of reconstituted eggs with ES cells in vitro and for obtaining young following transfer to recipients should be improved."

Also, Saito et al., (Id.) disclosed the use of "ES-like cell lines" (which were cultured over several passages) as nuclear donors in NT techniques. However, the resultant embryos only reached the 8- to 16-cell stage.

The only reported successes with NT (fused embryo upon implantation results in viable pregnancy) have been obtained by nuclear transfer techniques using blastocyst-derived inner cell mass (ICM) and cultured ICM cells as nuclear donors. (See e.g., Sims et al., "Production of fetuses from totipotent cultured bovine inner cell mass cells"; Theriogenology, 34, 313, 1993). See also, Keefer et al., Theriogenology, 39, 342, 1993 who report the use of bovine inner cell mass (ICM) cells derived from bovine expanded blastocysts as donor nuclear for the production of nuclear transfer embryos. The authors further disclose that the resultant embryos when implanted into maternal recipients result in apparently successful pregnancies (100 and 190 gestation at reference date).

Further, Collas et al., Biol. Reprod. 45, 455-465, 1991, disclose the use of rabbit cleavage-, morula- and blastocyst- embryonic cells as nuclear donors for NT. Also, Smith et al., Biol. Reprod., 40, 1027-1035, 1989, report the fusion of single cells derived from sheep 16-cell embryos, and the inner cell mass (ICM) of early blastocyst stage sheet embryos, with unfertilized enucleated secondary oocytes, the implantation of the resultant fused embryos into ewes, and the development of lambs which apparently express the phenotype of the nuclear donor breed.

However, while there are reported successes in the use of ICM cells as nuclear donors during NT for the production of NT embryos and offspring the use thereof is disadvantageous for at least several reasons. For example, in ungulates, the blastocyst inner cell mass (ICM) appears after the first round of differentiation which distinguishes the ICM cells from the cells of the trophectoderm. Moreover, cells of the trophectoderm, which constitute the majority of blastocyst cells, are not totipotent. Therefore, it is necessary when using ICM cells as nuclear donors to remove or otherwise separate the ICM cells from the trophectoderm. However, this has proven to be relatively difficult.

Further, and more importantly, the use of ICM cells as nuclear donors only results in a limited number of cells from any given embryo which are available for use as NT donor nuclei. As discussed supra, once the embryo has differentiated past a certain stage (generally around the blastocyst stage) the embryo contains a diminishing number of cells which are totipotent and which may be used as suitable NT donors. This of course greatly limits the number of genetically identical offspring that can be produced from a single embryo.

Therefore, the use of ES cells as nuclear donors for NT would prove highly beneficial since it would both avoid the need to separate totipotent from differentiated cells prior to NT, and moreover would provide an essentially limitless supply of genetically identical cells for NT.

One method for the preparation of ungulate ES cells suitable as nuclear donors for NT is described in Example 6 herein below.

The discussion thus far has stressed the importance of embryonic stem cells for the production of cloned animals which are genetically identical. This, of course, is of great importance, especially in the agricultural industry for the production of identical animals having specific desirable traits, e.g., enhanced milk production or increased size. However, another important application of embryonic stem cell cultures is for the production of transgenic embryonic cell lines. Such transgenic embryonic stem cell lines may be used for in vitro research, or may be used as nuclear or cell donors for nuclear transfer to obtain transgenic embryos which, in turn, may be used for implantation into maternal recipients to produce transgenic animals.

Transgenic animals have tremendous research and commercial potential. For example, animals may be produced which contain disease causing genes and used as animal models for drug research. Also, transgenic animals may be produced which contain genes providing for desirable traits, e.g., growth hormone genes.

The production of transgenic animals, and in particular transgenic ungulates, has been reported in the literature. For example, Simons et al., Bio/Technology, 6, 179-183, 1988, report the production of transgenic sheep by microinjection technique. Also, Rexroad et al., Mol. Reprod. Dev., 1, 164-169, 1989, report the integration of growth-regulating genes in sheep by microinjection of pronuclei.

Also, Biery et al., Theriogenology, 29(1), 224, 1988, disclose pronuclei injection of DNA into bovine zygotes; and McEvoy et al., Theriogenology, 33(4), 819-828, 1990, report the microinjection of very early stage embryos (one and two cell containing bovine ova) to create transgenic cattle. Further, Wilmut et al., Theriogenology, 33(1), 113-123, 1990, report gene transfer in order to modify milk production in bovines by the microinjection of bovine pronuclei with foreign DNA. The reference also discusses retroviral and stem cell transfer to blastocysts, and the use of sperm containing foreign DNA to fertilize eggs.

A significantly improved method to produce transgenic nonhuman animals, in particular bovines, entails microinjection of embryonic cells with desired genetic material, e.g., a homologous or heterologous DNA, and then use of the injected cells as nuclear donors for enucleated eggs. The injected cell and egg are fused, cultured to a requisite stage of development, and then implanted in a maternal animal for the production of genetically transformed animals. This method has been reported to provide for much greater efficiencies than pronuclear injection. Hill et al., Theriogenology, 37, 222, 1992, and Bondioli et al., "Transgenic Animals", First, N. and Haseltine F. (eds.), Butterworth-Heinemann, Stoncham, MA., pp. 265-273, 1991.

See also a recent review article pertaining to the production of transgenic farm animals. Pursel et al., "Status of Research with Transgenic Farm Animals", J. Animal Sci., 71, 10-19, 1993.

Another important application of ES cells is for the production of chimeric animals. Mouse ES cells have been used extensively for the production of chimeras. (See, e.g., Beddington et al., Development, 105, 733-737, 1989); Bradley et al., Nature, 309, 255-256, 1984; Lu et al., Proc. Natl. Acad. Sci., USA, 77, 6012-6016, 1980, 104, 175-182, 1988). However, to date methods for obtaining chimeric animals have not utilized an initial NT procedure. Instead, chimeras are typically produced by injecting 5 to 20 ES cells into a fertilized embryo which is at the morula stage (Tokunage et al., Devel. Growth and Different, 34, 561-566, 1992) or the blastocyst stage (Bradley et al., Nature, 309, 255-256, 1989). A chimeric offspring is only produced when the ES cells themselves contribute to the ICM. Moreover, the efficiencies of such methods appear to vary from report to report.

It is has been hypothesized by some researchers that these differences may be attributable to factors such as the strain of mouse used to produce the ES cells, the methods used for handling the culture, and the age of the culture. Also, one study suggested that smaller ES cells gave a higher percentage of chimeras when they were injected into the blastocyst stage embryo (Brown et al., In Vitro Cell Dev. Biol., 28A, pp. 773-778, 1992). Additionally, methods for producing chimeric offspring in mice have been improved by combining tetraploid mouse embryos with mouse ES cells, resulting in offspring that are almost completely (about 95%) derived from ES cells (Nagy et al., Development, 110(3), 815-821, 1990). However, the resultant offspring died within 48 hours of birth.

### Objects of the Invention

It is an object of the invention to provide a novel method of nuclear transplantation comprising introducing into a recipient oocyte a totipotent embryonic stem cell.

It is a more specific object of the invention to provide a novel method of nuclear transplantation comprising introducing into a recipient ungulate oocyte a totipotent ungulate embryonic stem cell.

It is an even more specific object of the invention to provide a novel method of nuclear transplantation comprising introducing into a recipient bovine oocyte a totipotent bovine embryonic stem cell.

It is another object of the invention to provide a method for producing chimeric non-human animals, preferably ungulates, by introducing one or more blastomeres, preferably from a similar staged fertilized embryo, into nuclear transfer embryos produced using embryonic stem cells as the donor nuclei.

It is another object of the invention to provide a method for producing transgenic non-human animals, preferably ungulates, by using transgenic embryonic stem cells as donor nuclei during nuclear transfer methods.

It is a more specific object of the invention to provide a novel method of nuclear transfer comprising the following steps:
(i) obtaining a non-human embryonic stem cell culture;
(ii) disaggregating the embryonic stem cell culture into individual cells;
(iii) introducing an individual embryonic stem cell into an enucleated recipient oocyte;
(iv) fusing the embryonic stem cell and the enucleated recipient oocyte to produce a fused nuclear transfer embryo;
(v) activating the fused nuclear transfer embryo; and
(vi) culturing the activated fused nuclear transfer embryo under conditions which provide for the embryo to attain a stage of development such that it may be implanted into a maternal recipient.

It is another specific object of the invention to provide a novel method for producing non-human chimeric embryos, preferably ungulate embryos, comprising the following steps:
(i) obtaining a non-human embryonic stem cell culture;
(ii) disaggregating the embryonic stem cell culture into individual cells;
(iii) introducing an individual disaggregated embryonic stem cell into an enucleated oocyte;
(iv) fusing the embryonic stem cell and enucleated oocyte to produce a fused nuclear transfer embryo;
(v) activating the fused nuclear transfer embryo;
(vi) culturing the fused nuclear transfer embryo;
(vii) introducing one or more blastomeres into the perivitelline space of the cultured nuclear transfer embryo; and
(viii) culturing the resultant chimeric embryo to a sufficient stage of development such that it may be successfully implanted into a maternal recipient and result in a viable pregnancy.

It is another object of the invention to provide a novel method of producing transgenic embryos comprising the following steps:
(i) obtaining a non-human embryonic stem cell culture;
(ii) disaggregating the embryonic stem cell culture into individual cells;
(iii) transfecting said individual cells with a desired polynucleotide and selecting for transgenic embryonic stem cells which comprise said polynucleotide stably integrated in their genome;
(iv) introducing a transgenic embryonic stem cell into an enucleated recipient oocyte;
(v) fusing the transgenic embryonic stem cell and enucleated recipient oocyte to produce a transgenic nuclear transfer embryo;
(vi) activating the transgenic nuclear transfer embryo; and
(vii) culturing the activated transgenic nuclear transfer embryo to a sufficient stage of development such that it may be implanted into a non-human maternal recipient and result in a viable pregnancy.

### Detailed Description of the Invention

The present invention is generally directed to a method for using embryonic stem (ES) cells as nuclear donors in nuclear transplantation (NT) procedures. As discussed supra, to the best knowledge of the present inventors there has been no previous report of the successful use of embryonic stem cells to produce nuclear transplantation fetuses or offspring. Rather, previously disclosed NT methods have used embryonic cells as donor nuclei, derived from embryos which range in size from about the cleavage stage to about the blastocyst stage. By contrast, the present invention provides for nuclear transfer embryos using embryonic stem (ES) cells as donor nuclei. This comprises a significant advance over previous nuclear transfer methods since embryonic stem (ES) cell lines may be obtained in essentially limitless numbers.

In general, nuclear transfer procedures comprise the transplantation of living nuclei from embryonic cells, or the whole embryonic cells themselves, into recipient cells, typically unfertilized eggs, followed by the fusion of the donor and recipient. By contrast, in the present invention, nuclear transfer will generally comprise the transplantation of a non-human embryonic stem cell into an enucleated oocyte, which cells are then fused to produce a nuclear transfer embryo.

Any non-human embryonic stem line which upon implantation into an enucleated oocyte and fusion results in a nuclear transfer embryo which provides for a viable pregnancy upon implantation into a maternal recipient is within the scope of the present invention. Preferably, however, ungulate embryonic stem cell cultures will be obtained and used as the donor nuclei. Methods for producing ungulate embryonic stem cell cultures, e.g., bovine and porcine embryonic stem cells have been described in the literature. See e.g., WO 90/06354; Saito et al., Roux's Arch. Dev. Biol., 201, 134-141, 1992; and Weima et al., Proc. 12th Int. Cong. Animal Reprod., 2, 766-768, 1992.

In the preferred embodiment, the embryonic stem cells will be produced according to the method described in Example 6, infra.

In general, ES cell lines will be produced as follows:
(i) removing the zona pellucida from a preblastocyst stage embryo;
(ii) disaggregating the cells of the preblastocyst stage embryo into individual blastomeric cells; and
(iii) culturing the resultant blastomeres in a culture medium which prevents differentiation of the blastomeres but which permits the formation of embryonic stem cell colonies.

In the most preferred embodiment, the culturing step will be effected by co-culturing the blastomeres on a feeder cell layer which prevents differentiation, preferably fibroblasts or buffalo rat liver cells until embryonic stem cell monolayers are obtained. Generally, when using bovine pre-blastocyst of stage embryo cells as the embryonic stem cell progenitors this occurs about every 7 to 10 days, at which time the embryonic stem cell monolayer should be passaged into another cell culture medium, preferably a culture comprising another feeder cell layer which prevents differentiation of the blastomeres. At about this time the ES cells form a monolayer and start to look "embryoid" in appearance.

Although Example 6 illustrates the production of embryonic stem cells derived from pre-blastocyst stage embryonic stem cells, the present invention is not limited to the use of embryonic stem cells derived from pre-blastocyst stage embryonic cells, but should be recognized by those skilled in the art as embracing the use of any embryonic stem cells which are suitable for use in the subject NT method. However, preferably ES cell lines will comprise embryonic stem cell lines produced from blastomeres obtained from embryos ranging from about the cleavage stage to about the blastocyst stage. Therefore, for ungulates, embryonic stem cell lines will be produced from blastomeres obtained from ungulate embryos ranging in size from about 2 to about 64 cells.

To obtain embryonic stem cells useful for nuclear transfer it is necessary to disaggregate ES cell cultures to provide for single ES cells. This may be effected by chemical or mechanical means, however, mechanical means are generally preferred. In addition, a pronase treatment at about 0.1 to 1.0 mg/ml may also be effected to facilitate breaking down the outer layers of the ES cells.

In the preferred embodiment, embryonic stem cells which are to be used for nuclear transfer will be cut out of a embryonic stem cell monolayer comprised on a suitable feeder layer, preferably fibroblasts, and lifted off the feeder layer using a glass needle. The resultant mass of embryonic stem cells will then be incubated in a suitable disaggregation medium, e.g., a HEPES buffered medium containing from about 0.1 to 1.0 mg/ml of pronase. The cells thus cultured will be under conditions which provide for disaggregation of the ES cells. Culturing for about one to four hours at 39°C in a pronase containing HEPES medium has been found to provide for suitable disaggregation. However, the time and disaggregation medium may be varied dependent upon, e.g., the age of the embryonic stem cell culture, and whether it is derived from pre-blastocyst or blastocyst embryonic cells, among other factors.

After the cells have been disaggregated into a single cell suspension they may be used in nuclear transfer procedures. Preferably the embryonic stem cells will be used within about 24 hours after disaggregation since the available data suggests that NT embryonic developmental rates decrease when the embryonic stem cells are used for nuclear transfer after this time.

The resultant embryonic stem cells, preferably ungulate stem cells, and most preferably bovine embryonic stem cells will then be introduced into suitable recipient enucleated oocytes. For the successful commercial use of techniques such as genetic engineering, nuclear transfer and cloning, oocytes must generally be matured in vitro before these cells may be used as recipient cells for nuclear transfer, and before they can be fertilized by the sperm cell to develop into an embryo. This process generally requires collecting immature (prophase I) oocytes from bovine ovaries obtained at a slaughterhouse and maturing the oocytes in a maturation medium prior to fertilization or enucleation until the oocyte attains the metaphase II stage, which generally occurs about 18-24 hours post-aspiration. For purposes of the present invention, this period of time is known as the "maturation period." As used herein for calculation of time periods, "aspiration" refers to aspiration of the immature oocyte from ovarian follicles.

Additionally, metaphase I stage oocytes, which have been matured in vivo have been successfully used in nuclear transfer techniques. Essentially, mature metaphase II oocytes are collected surgically from either non-superovulated or superovulated cows or heifers 35 to 48 hours past the onset of estrus or past the injection of human chorionic gonadotropin (hCG) or similar hormone.

The stage of maturation of the oocyte at enucleation and nuclear transfer has been reported to be significant in the efficacy of NT methods. (See e.g., Prather, First and Differentiation, 48, 1-8, 1991). In general, successful mammalian embryo cloning practices use the metaphase II stage oocyte as the recipient oocyte because at this stage it is believed that the oocyte is sufficiently "activated" to treat the introduced nucleus as it does a fertilizing sperm. In domestic animals, and especially cattle, the oocyte activation period generally ranges from about 16-52 hours, preferably about 28-42 hours post-aspiration.

For example, immature oocytes may be washed in HEPES buffered hamster embryo culture medium (HECM) as described in Seshagine et al., Biol. Reprod., 40, 544-606, 1989 and then placed into drops of maturation medium consisting of 50 microliters of tissue culture medium (TCM) 199 containing 10% fetal calf serum which contains appropriate gonadotropins such as leutenizing hormone (LH) and follicle stimulating hormone (FSH), and estradiol under a layer of lightweight paraffin or silicon at 39°C.

After a fixed time maturation period, which ranges from about 10 to 40 hours, and preferably about 16 hours, the oocytes will be enucleated. However, prior to enucleation the oocytes will preferably be removed and placed in HECM containing 1 milligram per milliliter of hyaluronidase prior to removal of cumulus cells. This may be effected by repeated pipetting through very fine bore pipettes or by vortexing briefly. The stripped oocytes are then screened for polar bodies, and the selected metaphase II oocytes, as determined by the presence of polar bodies, are then used for nuclear transfer. Enucleation follows.

Enucleation may be effected by known methods, such as described in U.S. Patent No. 4,994,384. Essentially, metaphase II oocytes are either placed in HECM, optionally containing 7.5 micrograms per milliliter cytochalasin B, for immediate enucleation, or may be placed in a suitable medium, for example CRlaa, plus 10% estrus cow serum, and then enucleated later, preferably not more than 24 hours later, and more preferably 16-18 hours later.

Enucleation may be accomplished microsurgically using a micropipette to remove the polar body and the adjacent cytoplasm. The oocytes may then be screened to identify those of which have been successfully enucleated. This screening may be effected by staining the oocytes with 1 microgram per milliliter 33342 Hoecht dye in HECM, and then viewing the oocytes under ultraviolet irradiation for less than 10 seconds. The oocytes that have been successfully enucleated can then be placed in a suitable culture medium, e.g., CRlaa plus 10% serum.

In the present invention, the recipient oocytes will preferably be enucleated at a time ranging from about 10 hours to about 40 hours after the initiation of in vitro maturation, more preferably from about 16 hours to about 24 hours after initiation of in vitro maturation, and most preferably about 16 hours after initiation of in vitro maturation.

A single embryonic stem cell will then be transferred into the perivitelline space of the enucleated oocyte. The embryonic stem cell and enucleated oocyte will then preferably be fused by methods which are known in the art. For example, the cells may be fused by electrofusion. Electrofusion is accomplished by providing a pulse of electricity that is sufficient to cause a transient breakdown of the plasma membrane. This breakdown of the plasma membrane is very short because the membrane reforms rapidly. Essentially, if two adjacent membranes are induced to breakdown and upon reformation the lipid bilayers intermingle, small channels will open between the two cells. Due to the thermodynamic instability of such a small opening, it enlarges until the two cells become one. See U.S. Patent 4,997,384 by Prather et al., for a further discussion of this process. A variety of electrofusion media can be used including e.g., sucrose, mannitol, sorbitol and phosphate buffered solution. Fusion can also be accomplished using Sendai virus as a fusogenic agent (Graham, Wister Inot. Symp. Monogr. 9, 19, 1969).

Preferably, the ES cell and oocyte are electrofused in a 500 *µ*m chamber by application of an electrical pulse of 90V for 15 *µ*sec.

After fusion, the resultant embryonic stem cell nuclear transfer embryo will then be "activated" by placing the fused embryo in an activation medium. Table 1, infra, illustrates that the oocyte has to be activated prior to fusion in order to obtain a high developmental rate when using non-synchronized blastomeres obtained from embryos as nuclear transfer donors. In particular, it was found that developmental rates to morula and blastocyst stage for blastomere derived NT embryos improved significantly when fusion was induced after, rather than before, the time of activation.

Surprisingly, the present inventors have discovered that the reverse is true for ES cell derived NT embryos. Instead, developmental rates for ES cell NT embryos are highest when fusion occurs prior to activation. This was demonstrated by varying the time of fusion from about 14 hours prior to activation to about 9 hours after activation as illustrated in Example 2, infra. This is a fundamental difference between embryo and ES cell NT procedures.

The fused NT embryos may be activated by a variety of methods. Such methods include, e.g., culturing the NT embryo at sub-physiological temperature, in essence by applying a cold, or actually cool temperature shock to the NT embryo. This may be most conveniently done by culturing the NT embryo at room temperature, which is cold relative to the physiological temperature conditions to which embryos are normally exposed.

Alternatively, activation may be achieved by known activation agents. For example, penetration of oocytes by sperm during fertilization has been shown to activate prefusion oocytes to yield greater numbers of viable pregnancies and multiple genetically identical calves after nuclear transfer. Also, treatments such as electrical and chemical shock may be used to activate NT embryos after fusion.

Additionally, activation may be effected by:
(i) increasing levels of divalent cations in the oocyte, and
(ii) reducing phosphorylation of cellular proteins in the oocyte.
This will generally be effected by introducing divalent cations into the oocyte cytoplasm, e.g., magnesium, strontium, barium or calcium, e.g., in the form of an ionophore. Other methods of increasing divalent cation levels include the use of electric shock, treatment with ethanol and treatment with caged chelators.

Phosphorylation may be reduced by known methods, e.g., by the addition of kinase inhibitors, e.g., serine-threonine kinase inhibitors, such as 6-dimethylaminopurine, staurosporine, 2-aminopurine, and sphingosine.

Alternatively, phosphorylation of cellular proteins may be inhibited by introduction of a phosphatase into the oocyte, e.g., phosphatase 2A and phosphatase 2B.

In the preferred embodiment, NT activation will be effected by briefly exposing the fused ES derived NT embryo to a HECM containing medium containing 5*µ*M ionomycin and 1 mg/ml BSA, followed by a 5 minute dilution into HECM containing 30 mg/ml BSA within about 24 hours after fusion, and preferably about 4 to 9 hours after fusion.

The activated NT embryos may then be cultured in a suitable in vitro culture medium. Culture media suitable for culturing and maturation of embryos are well known in the art. Examples of known media, which may be used for bovine embryo culture and maintenance, include Ham's F-10 + 10% fetal calf serum (FCS), Tissue Culture Medium-199 (TCM-199) + 10% fetal calf serum, Tyrodes-Albumin-Lactate-Pyruvate (TALP), Dulbecco's Phosphate Buffered Saline (PBS), Eagle's and Whitten's media. One of the most common media used for the collection and maturation of oocytes is TCM-199, and 1 to 20% serum supplement including fetal calf serum, newborn serum, estrual cow serum, lamb serum or steer serum. A preferred maintenance medium includes TCM-199 with Earl salts, 10% fetal calf serum, 0.2 MM Ma pyruvate and 50 *µ*g/ml gentamicin sulphate. Any of the above may also involve co-culture with a variety of cell types such as granulosa cells, oviduct cells, BRL cells and uterine cells.

Another maintenance medium is described in U.S. Patent 5,096,822 to Rosenkrans, Jr. et al.. This embryo medium, named CR1, contains the nutritional substances necessary to support an embryo.

CR1 contains hemicalcium L-lactate in amounts ranging from 1.0 mM to 10 mM, preferably 1.0 mM to 5.0 mM. Hemicalcium L-lactate is L-lactate with a hemicalcium salt incorporated thereon. Hemicalcium L-lactate is significant in that a single component satisfies two major requirements in the culture medium: (i) the calcium requirement necessary for compaction and cytoskeleton arrangement; and (ii) the lactate requirement necessary for metabolism and electron transport. Hemicalcium L-lactate also serves as valuable mineral and energy source for the medium necessary for viability of the embryos.

Advantageously, CR1 medium does not contain serum, such as fetal calf serum, and does not require the use of a co-culture of animal cells or other biological media, i.e., media comprising animal cells such as oviductal cells. Biological media can sometimes be disadvantageous in that they may contain micro-organisms or trace factors which may be harmful to the embryos and which are difficult to detect, characterize and eliminate.

Examples of the main components in CR1 medium include hemicalcium L-lactate, sodium chloride, potassium chloride, sodium bicarbonate and a minor amount of fatty-acid free bovine serum albumin (Sigma A-6003). Additionally, a defined quantity of essential and non-essential amino acids may be added to the medium. CR1 with amino acids is known by the abbreviation "CR1aa."

CR1 medium preferably contains the following components in the following quantities:

| | |
|---|---|
| sodium chloride | 114.7 mM |
| potassium chloride | 3.1 mM |
| sodium bicarbonate | 26.2 mM |
| hemicalcium L-lactate | 5 mM |
| fatty-acid free BSA | 3 mg/ml |

In the preferred embodiment, after activation the NT embryos will be placed in CRlaa medium containing 1.9 mM DMAP for 4 hours followed by a wash in HECM and then cultured in CR1aa containing BSA.

After about three to four days the embryos may then be switched to CRlaa containing 10% fetal calf serum and cultured therein for the balance of their in vitro culture period. The resultant embryos may be nonsurgically transferred into recipient females anywhere from 5 to 9 days after the time of activation. However, this is only exemplary. Variation of the culture medium and culturing methods is within the level of skill in the art.

An alternative utility of the above-described ES derived NT embryos is for the production of chimeric embryos. As discussed previously, methods of using ES cells to produce chimeric embryos are generally known. However, methods to date have not combined these techniques with NT. The present inventors have developed a novel method of producing chimeric embryos whereby ES derived NT embryos are used as the embryonic recipients to which are introduced blastomeres from a embryo, preferably a similar staged fertilized embryo.

Essentially, this method will comprise the following steps:
(i) producing an ES cell derived NT embryo as described supra;
(ii) artificially activating the NT embryo;
(iii) culturing the cultivated NT embryo to a desired cell stage;
(iv) introducing one or more blastomeres from an embryo into the perivitelline space of the NT embryo and
(v) culturing the resultant chimeric embryo until the late morula or blastocyst stage of development so that it may be implanted into a maternal recipient.

Preferably the activation step (ii) will be effected as described previously, i.e. by a 4 minute exposure to 5*µ*M ionomycin in HECM medium containing 1 mg/ml BSA followed by a 5 minute dilution into HECM containing 30 mg/ml BSA.

The culturing step (iii) will be effected by known methods for culturing NT embryos and may be effected in vitro or in vivo. Preferably, culturing will be effected in vitro in CRlaa medium containing 1.0 mM DMAP for 4 hours followed by a wash in HECM, which is in turn followed by culturing in CRlaa containing BSA until the embryo reaches the desired cell stage. Preferably, this cell stage will range from about the cleavage stage to about the blastocyst cell stage which ranges from about 2 cells to about 64 cells into embryonic development. Most preferably, the NT embryos will be cultured until they develop into about 8 and 16-cell embryos.

Step (iv) will comprise the introduction of one or more blastomeres into the perivitelline space of the NT embryo which has been cultured to the desired cell stage. Preferably, about two blastomeres from a similar staged fertilized embryo will be placed in the perivitelline space of the NT embryo. However, the number of blastomeres may vary from one to about ten cells. The blastomeres which are transferred into the ES cell derived NT embryos may comprise blastomeres obtained from embryos ranging in size from about the cleavage stage to about the blastocyst stage.

In the preferred embodiment the blastomeres which are introduced will comprise tetraploid cells since it has been previously shown in the mouse that tetraploid cells when used to produce chimeras preferentially contribute to the fetal placenta (Nagy et al., Development, 110(3), 815-821, 1990). Tetraploid embryos may be obtained by electrolyzing two-cell stage embryos resulting in one-cell stage embryos. Experiments conducted by the present inventors have demonstrated that when tetraploid cells are used as the introduced blastomeres it provides for around 50% of the resultant fetal tissue cells comprising cells derived from the ES cell clone.

After the chimeric embryo is produced it will then be cultured by known in vitro or in vivo methods prior to introduction into a maternal recipient where it will then develop into a viable chimeric offspring.

As discussed supra, the present invention further provides a novel method for producing transgenic embryos and offspring by nuclear transfer using transgenic embryonic stem cells as nuclear donors. In the preferred embodiment, transgenic ungulate animals, and most preferably transgenic bovines will be produced.

This will essentially comprise introduction of a desired polynucleotide into an embryonic stem cell, i.e., a particular DNA and/or RNA molecule, and the use of the resultant transgenic embryonic stem cell as a nuclear donor. Methods for the introduction of polynucleotide into cells in culture are well known in the art. Such methods include, e.g., electroporation, retroviral vector infection, particle acceleration, transfection, and microinjection.

The cells which contain the desired polynucleotide, e.g., a desired gene, which may be homologous or heterologous to the host cell, will then be selected according to known methods. For example, the cells may be co-transfected with a marker gene which provides for selection.

Once a culture of transgenic ES cells is obtained which contains a desired polynucleotide integrated into its genome, the individual cells may be used as NT donors in the above-described method. This is a particularly advantageous use of the present invention. Thereby, the transgenic ES cell will facilitate the production of many identical animals having an identical genetic modification, e.g., a desired gene integration. These animals will provide a means for in vivo study of the effects of the particular gene. Also, animals may be obtained which express a disease causing gene and used to assay potential drug therapies for treatment of the particular disease condition.

Moreover, transfection of ES cells in vitro with a desired polynucleotide will permit most of the genetic modification steps to be effected in vitro. This avoids the need to perform repeated breeding to introduce a desired gene into a particular genetic background. Also, one is able to characterize the donor cell genome in vitro (e.g., by use of suitable probes) that is not feasible when the donor is a primary embryo cell. Consequently, this procedure may be accomplished faster than previous breeding methods. Also, since one has a high expectation that the offspring will express the desired gene, the need to screen animals is substantially reduced. Therefore, the invention should significantly reduce the cost of animal breeding, and the production of transgenic animals, particularly among larger domesticated animals, which have relatively prolonged gestation periods and which bear only one or a few offspring per pregnancy.

Additionally, genetically altered NT embryos obtained using genetically altered ES cells as the nuclear donor may themselves be used as a source of blastomeres which are then introduced into the perivitelline space of ES derived NT embryos for the production of transgenic chimeric embryos.

The present invention will now be further illustrated by reference to the following examples which are provided solely for purposes of illustration and are not intended to be in any way limitative.

### Example 1

Essentially, the following example sets forth the preferred method for producing ES cell derived NT embryos. Oocytes are in vitro matured for about 16 hours followed by microsurgical enucleation by known methods such as are described supra. A single ES cell obtained by disaggregation of an ES cell culture is then transferred into the perivitelline space of the enucleated oocyte.

The ES cell is then fused with the enucleated oocyte by electrofusion in a 500 *µ*m chamber by application of an electrical pulse of 90 v for 15 *µ*sec. After fusion, the resultant ES derived NT embryo is then activated by a 4 minute exposure to 5 *µ*M ionomycin in HECM medium containing 30 mg/ml BSA. The embryos are then cultured in CRlaa medium containing 1.0 mM DMAP for 4 hours followed by washing in HECM followed by culturing in CRlaa containing BSA.

About three to four days after culturing the embryos are then switched to CRlaa containing 10% fetal calf serum for the remainder of the in vitro culture period. The embryos are then nonsurgically transferred into recipient females at about 5 to 9 days after activation.

### Example 2

In this example, the time in which the ES cell was fused into the enucleated oocyte was altered. The time of activation remained constant at 24 hrs after the initiation of oocyte maturation. The time of fusion ranged from 14 hrs prior to activation to 9 hrs after the time of activation. For purposes of comparison, at a number of these time points, 32-cell stage blastomeres were also used as donor nuclei. Blastomeres were isolated and transferred into recipient oocytes by the methods described supra.

Table 1 shows that developmental rates to morula and blastocyst stages differed depending on the type of donor nuclei used and the time of fusion. Developmental rates to morula and blastocyst stage for blastomere derived NT embryos were improved when fusion was induced after the time of activation; whereas, developmental rates for ES cell NT embryos were highest when fusion was prior to activation. As discussed supra, this is a fundamental difference between embryo and ES cell NT procedures.

**TABLE 1.**

| Effect of time of fusion with respect to activation on the developmental rate to morula and blastocyst stages Time of Fusion* | | | | | | | | |
|---|---|---|---|---|---|---|---|---|
| Donor Nuclei Source | -14 | -9 | -7 | -4 | -1 | +1 | +5 | +9 |
| Blastomere | | | | 10/74 0% | | 7/105 7% | 62/475 13% | 13/80 16% |
| ES Cell | 0/243 | 59/602 | 10% ↕ | 103/1045 | 13/549 | 16/526 | 47/2195 | 8/421 |
| | 0% | 10% | | 10% | 2% | 3% | 2% | 2% |

| | | | | | | | | |
|---|---|---|---|---|---|---|---|---|
| * Number of hours fusion pulse was prior to or after the time of activation (Example-1 = fusion pulse given 1 hour prior to activation) | | | | | | | | |
| ↕ Averaged % developmental rate from two treatments within the same time point | | | | | | | | |

A number of pregnancies were established with ES cell NT embryos, however, to date all of these have aborted prior to 60 days of pregnancy. Interestingly, the longest pregnancy was 35 days when the oocyte was activated prior to fusion, while two pregnancies of 50 to 55 days were obtained when fusion was induced prior to activation. Thus, it appears that the ES cell was more easily or more completely remodeled when it was exposed to the oocyte's cytoplasm for a period of time prior to activation.

### Example 3

In this example the time of fusion remained constant and the time of activation was altered. All of the ES cell NT embryos were fused at about 20 hours after the initiation of maturation. In the first group (AC24) the ES cell NT embryos were activated 4 hrs later at 24 hours after the initiation of maturation using the ionomycin and DMAP protocol described above. The second group (AC39) was not activated until 19 hours later using ionomycin and DMAP. This second group was held in CRlaa during the 19 hour waiting period. The results in table 2 shows that altering the time of activation as much as 19 hours after fusion does not significantly affect developmental rates to morula and blastocyst stage.

**TABLE 2.**

| The effect of time of fusion on developmental rates for ES cell NT embryos | | | |
|---|---|---|---|
| Treatment | No. NTs To Culture | No. NTs to Mor and Blast | Developmental Rate |
| ES Cell fused 4 hrs before Act. (AC24) | 1045 | 103 | 10% |
| ES Cell fused 19 hrs. before Act (AC39) | 568 | 43 | 8% |

### Example 4

Isolated single cell suspension ES cells range from 15 *µ*m to 21 *µ*m in cell diameter. With this diversity in size, there could be a subpopulation of ES cells that have a greater chance of developing into an embryo. Therefore, NT developmental rates to morula and blastocyst stages were monitored for different size donor ES cells. In this experiment the time of fusion and activation remained constant. Fusion was induced at 20 hrs and activation at 24 hrs after the initiation of maturation. The results of this experiment are provided in table 3. These data show that the smaller ES cell group has higher developmental rates to morula and blastocyst stage than the two other groups of ES cells with larger cell diameter. The reasons for these results may be attributable to the genetic makeup and/or the cell stage of the donor nucleus.

**TABLE 3.**

| The effect of ES cell diameter (15 *µ*m, 18 *µ*m and 21 *µ*m) on developmental rates of ES cell NT embryos | | | |
|---|---|---|---|
| Treatment | No. NTs to Culture | No. NTs to Mor & Blast | Developmental Rate |
| ES15 | 593 | 117 | 20% |
| ES18 | 703 | 88 | 13% |
| ES21 | 642 | 67 | 10% |

### Example 5

As previously mentioned, an ES cell NT embryo when transferred into a recipient animal will implant and differentiate into a fetus. Several fetuses have been shown to have beating hearts when examined through ultrasound visualization. However, the heartbeat has always been lost before sixty days of gestation. The cause for this loss was unknown since there have been no gross anatomical abnormalities observed.

In order to overcome these losses, chimeric embryos were produced. This procedure entails the transfer of two blastomeres from an eight to 16-cell stage fertilized embryo into the perivitelline space of an eight to 16-cell stage ES cell clone. The chimeric embryo is allowed to develop to the blastocyst stage and then is transferred to a recipient animal.

There were two types of blastomeres transferred into the ES cell clones. One was the normal diploid blastomeres and the other being blastomeres from a presumably tetraploid embryo. Tetraploid embryos were produced by electrofusing two-cell stage embryos resulting in one-cell stage embryos. The fused tetraploid embryos were then cultured in vitro until they reached the 8- to 16- cell stage at which time blastomeres from these embryos were used to produce chimeric embryos. Tetraploid cells were used because it has been shown in the mouse that when tetraploid cells were used to make chimeras these cells preferentially contribute to the placenta of the fetus (Nagy et al., 1992). When these cells were combined with entirely mouse ES cells, less than 5% of the cells in the resulting fetus and greater than 95% of the cells in the placenta were derived from the tetraploid cells.

The pregnancy results from these studies are presented in table 4. At this time five of the eleven on-going pregnancies have already passed the mid-point of gestation. Three of these pregnancies are presumably from tetraploid chimeras and two from diploid chimeras. Tissue samples from a tetraploid fetus that was taken on day 85 of gestation showed that around 50% of the cells in three different tissues were indeed derived from the ES cell clone.

**TABLE 4.**

| Pregnancy rates for ES cell clone chimeras | | | |
|---|---|---|---|
| Treatment | No. Embryos Transferred | No. Pregnancies Initiated (%) | No. Pregnancies On-Going (> 30 days) |
| Diploid Chimeras | 109 | 13 | 7 |
| Tetraploid Chimeras | 82 | 14 | 4 |

### Example 6

In this example a line of embryonic stem cells are produced. Preblastocyst stage bovine embryos were obtained from in vivo fertilizations and in vitro fertilizations. These embryos ranged in size from the 8-cell stage to the 64-cell stage, and were selected such that they were between 2 and 5 days from time of fertilization. These embryos were then treated with 1 mg/ml of pronase to remove the zona pellucida. Some of these embryos were then placed in phosphate buffered saline (PBS) medium containing 7.5 *µ*g/ml of cytochalasin B to reduce the compactness of the embryo structure and therefore facilitate the disaggregation of cells. However, some embryos were disaggregated without a cytochalasin B pretreatment.

Individual blastomeres, removed from the preblastocyst stage embryos ranging in size from between 8 to 64 cells, were then placed either on top or underneath a mouse fibroblast feeder cell layer prepared according to the method described infra. In particular, the feeder cell layer was prepared from mouse fetuses at ten to twenty days of gestation. The head, liver, heart and alimentary tract were removed from the fetuses and the remaining tissue was washed and incubated at 37°C in 0.05% trypsin-0.02% EDTA. Loose cells were then cultured in tissue culture flasks containing MEM-α supplemented with penicillin, streptomycin, 10% fetal calf serum and 0.1 mM B mercaptoethanol. The feeder cell cultures were established over a two- to three-week culture period at 37°C, 5% CO₂ and at 100% humidity. These cells were then treated with mitomycin C at 10 *µ*g/ml prior to their usage as feeder cells.

The mitomycin C-pretreated fibroblast layer was then used as a feeder cell layer for the blastomeres. In some experiments the individual blastomeres were placed on top of the feeder cell layer. However, ES cell lines were more readily established, and differentiation was better inhibited, when the blastomeres were placed beneath the feeder layer. It is believed that this enhanced cell-to-cell contact provides for the blastomeres to be more in contact with membrane associated differentiating inhibiting factors such as leukemia inhibiting factor (LIF). Cells placed on top of the feeder layer had lesser cell-to-cell contact between the blastomeres and feeder cells. Also, the blastomeres occasionally differentiated into trophoblast vesicles.

Every two to three days, the MEM-α plus 10% FCS growth medium was replaced. After the cells had been cultured for a total of about seven to ten days, embryonic stem cell monolayers were obtained. Also, around this time into the culturing, the blastomeres (which now had become ES cell lines) start to differentiate and exhibit an embryoid-like appearance.

Accordingly, the cells are passaged at this time onto new feeder layers. This was effected mechanically using a fine glass needle to cut the ES cell monolayer into smaller cell clusters. These cell clusters were then repeatedly pipetted at a 1:100 dilution onto fresh fibroblast feeder layers.

This method has resulted in the generation of numerous ES cell lines from preblastocyst-derived embryos from the 8- to 64-cell stage, and has provided for both male and female ES cell lines.

The ES cell data obtained by the above-described method is presented in Table 5 below.

**TABLE 5**

| **EMBRYONIC STEM CELL DATA** | | | |
|---|---|---|---|
| Starting Embryo | No. Starting Embryos | No. ES Cell Lines | Efficiency |
| Blastocyst | 42 | 22 | 53% |
| Morula | 11 | 6 | 55% |
| 8-16 Cell | 12 | 8 | 67% |
| 4-cell | 30 | 0 | 0% |

## Claims

1. An ungulate embryonic stem cell culture, produced by the method comprising:
(i) removing the zona pellucida from an embryo having between 2 and 64 cells;
(ii) disaggregating the cells of the embryo into individual blastomeric cells; and
(iii) culturing the resultant blastomeres in a culture medium that prevents differentiation of the blastomeres but that permits the formation of embryonic stem cell colonies.

2. The ungulate embryonic stem cell culture of claim 1, wherein said blastomeres are co-cultured with a feeder cell layer.

3. The ungulate embryonic stem cell culture of claim 2, wherein the feeder cell layer comprises fibroblasts.

4. The ungulate embryonic stem cell culture of claim 3, wherein said fibroblasts are mouse fetal fibroblasts.

5. The ungulate embryonic stem cell culture of claim 2, wherein said blastocysts are placed between the feeder cell layer and the culture dish.

6. The ungulate embryonic stem cell culture of claim 1, wherein said embryo is a preblastocyst stage embryo.

7. The ungulate embryonic stem cell culture of claim 1, wherein said embryo is an 8-cell to 64-cell embryo.

8. The ungulate embryonic stem cell culture of claim 1, wherein the method further comprises passaging the cultured cells of step (iii) one or more times.

9. The ungulate embryonic stem cell culture of claim 8, wherein the cell culture comprises cells having a diameter of between about 15 µm and about 21 µm.

10. The ungulate embryonic stem cell culture of claim 1, wherein the method further comprises disaggregating one or more of the cultured cells of step (iii) into a suspension comprising single cells.

11. The ungulate embryonic stem cell culture of claim 1, wherein said culture comprises transgenic cells.
